# EUROPEAN PATENT APPLICATION

(11) **EP 2 031 061 A2**
(43) Date of publication of application: **04.03.2009**
(21) Application number: 08075726.3
(22) Date of filing: 22.08.2008
(51) Int. Cl.: C12N 15/11, A61K 31/713

(54) **siDNA against influenza virus**

(30) Priority: 03.09.2007 EP 07075751
(71) Applicant: Mölling, Karin, 14195 Berlin (DE)
(72) Inventor: Mölling, Karin, 14195 Berlin (DE)
(74) Representative: Boeckh, Tobias

(57) **Abstract**

Silencing of Influenza virus RNA can be achieved by siDNA. These are oligodeoxynucleotides consisting of an antisense-strand homologous to the viral RNA and a second strand, partially complementary to the antisense-strand. The two strands are preferentially linked by a linker (eg 4 thymidines). Triple-helix formation with the target RNA is a preferred effect. The siDNA is superior to siRNA because it acts earlier, is easier taken up by the cell, the formation of RNA-DNA hybrids is preferred over double-stranded DNA or double-stranded RNA, which forms as tertiary structures in RNA genomes. Also the induction of interferon is less likely. siDNA is easier to synthesize and it is more stable. It can be combined with siRNA.

SEQUENCE LISTING

<110>  Moelling, Karin

<120>  siDNA against Influenza Virus

<130>  XI 971/08

<150>  EP 07075751.3
<151>  2007-09-03

<160>  15

<170>  PatentIn version 3.5

<210>  1
<211>  24
<212>  RNA
<213>  artificial

<220>
<223>  target sequence (RNA)

<400>  1
aaacaaugaa agaguauggg gagg                                              24


<210>  2
<211>  25
<212>  DNA
<213>  artificial

<220>
<223>  ODN IPA5' (siDNA) - antisense

<400>  2
   ttttttgcta agagttgggg ctgga                                              25


<210>  3
<211>  25
<212>  DNA
<213>  artificial

<220>
<223>  ODN IPA5' (siDNA) - second strand

<400>  3
tcctccccat actctttcat tgttt                                             25


<210>  4
<211>  25
<212>  RNA
<213>  artificial

<220>
<223>  Target Sequence PPT (RNA)

<400>  4
aaaaugaaau ggggaaugga gauga                                             25


<210>  5
<211>  25
<212>  DNA
<213>  artificial

<220>
<223>  ODN IPA-PPT (siDNA) - antisense

<400>  5
ttttgctttt ggggatggga cttga                                          25


<210>  6
<211>  25
<212>  DNA
<213>  artificial

<220>
<223>  ODN IPA-PPT (siDNA) - second strand

<400>  6
tcatctccat tccccatttc atttt                                          25


<210>  7
<211>  25
<212>  RNA
<213>  artificial

<220>
<223>  Target Sequence si (RNA)

<400>  7
uaugaagcaa uugaggagug ccuga                                          25


<210>  8
<211>  25
<212>  DNA
<213>  artificial

<220>
<223>  ODN IPAsi (siDNA)- antisense

<400>  8
gtgcttctta ttgagctgtg tttga                                          25


<210>  9
<211>  25
<212>  DNA
<213>  artificial

<220>
<223>  ODN IPAsi (siDNA)- second strand

<400>  9
tcaggcactc ctcaattgct tcata                                          25


<210>  10
<211>  2233
<212>  DNA
<213>  Influenza A virus segment 3, polymerase PA

<400>  10
agcgaaagca ggtactgatc caaaatggaa gattttgtgc gacaatgctt caatccgatg      60

attgtcgagc ttgcggaaaa aacaatgaaa gagtatgggg aggacctgaa aatcgaaaca     120

aacaaatttg cagcaatatg cactcacttg gaagtatgct tcatgtattc agattttcac     180

ttcatcaatg agcaaggcga gtcaataatc gtagaacttg gtgatccaaa tgcacttttg     240

aagcacagat ttgaaataat cgagggaaga gatcgcacaa tggcctggac agtagtaaac 300
agtatttgca acactacagg ggctgagaaa ccaaagtttc taccagattt gtatgattac 360
aaggagaata gattcatcga aattggagta acaaggagag aagttcacat atactatctg 420
gaaaaggcca ataaaattaa atctgagaaa acacacatcc acattttctc gttcactggg 480
gaagaaatgg ccacaaaggc agactacact ctcgatgaag aaagcagggc taggatcaaa 540
accagactat tcaccataag acaagaaatg gccagcagag gcctctggga ttcctttcgt 600
cagtccgaga gaggagaaga gacaattgaa gaaaggtttg aaatcacagg aacaatgcgc 660
aagcttgccg accaaagtct cccgccgaac ttctccagcc ttgaaaattt tagagcctat 720
gtggatggat tcgaaccgaa cggctacatt gagggcaagc tgtctcaaat gtccaaagaa 780
gtaaatgcta gaattgaacc ttttttgaaa acaacaccac gaccacttag acttccgaat 840
gggcctccct gttctcagcg gtccaaattc ctactgatgg atgccttaaa attaagcatt 900
gaggacccaa gtcatgaagg agagggaata ccgctatatg atgcaatcaa atgcatgaga 960
acattctttg gatggaagga acccaatgtt gttaaaccac acgaaaaggg aataaatcca 1020
aattatcttc tgtcatggaa gcaagtactg gcagaactgc aggacattga gaatgaggag 1080
aaaattccaa agactaaaaa tatgaagaaa acaagtcagc taaagtgggc acttggtgag 1140
aacatggcac cagaaaaggt agactttgac gactgtaaag atgtaggtga tttgaagcaa 1200
tatgatagtg atgaaccaga attgaggtcg cttgcaagtt ggattcagaa tgagtttaac 1260
aaggcatgcg aactgacaga ttcaagctgg atagagctcg atgagattgg agaagatgtg 1320
gctccaattg aacacattgc aagcatgaga aggaattatt tcacatcaga ggtgtctcac 1380
tgcagagcca cagaatacat aatgaagggg gtgtacatca atactgcctt gcttaatgca 1440
tcttgtgcag caatggatga tttccaatta attccaatga taagcaagtg tagaactaag 1500
gagggaaggc gaaagaccaa cttgtatggt ttcatcataa aaggaagatc ccacttaagg 1560
aatgacaccg acgtggtaaa ctttgtgagc atggagtttt ctctcactga cccaagactt 1620
gaaccacata aatgggagaa gtactgtgtt cttgagatag gagatatgct tataagaagt 1680
gccataggcc aggtttcaag gcccatgttc ttgtatgtga gaacaaatgg aacctcaaaa 1740
attaaaatga aatggggaat ggagatgagg cgttgcctcc tccagtcact tcaacaaatt 1800
gagagtatga ttgaagctga gtcctctgtc aaagagaaag acatgaccaa agagttcttt 1860
gagaacaaat cagaaacatg gcccattgga gagtccccca aaggagtgga ggaaagttcc 1920
attgggaagg tctgcaggac tttattagca aagtcggtat tcaacagctt gtatgcatct 1980
ccacaactag aaggattttc agctgaatca agaaaactgc ttcttatcgt tcaggctctt 2040
agggacaacc ttgaacctgg gacctttgat cttggggggc tatatgaagc aattgaggag 2100
tgcctgatta atgatccctg ggttttgctt aatgcttctt ggttcaactc attccttaca 2160
catgcattga gttagttgtg gcagtgctac tatttgctat ccatactgtc caaaaaagta 2220
ccttgtttct act 2233

<210> 11
<211> 2233
<212> DNA
<213> Polymerase A (PA) of Influenza H5N1

<400> 11
agcaaaagca ggtactgatc caaaatggaa gactttgtgc gacaatgctt caatccaatg 60

attgtcgagc ttgcggaaaa gacaatgaaa gagtatgggg aagatccgaa gattgaaaca 120

aacaagttcg ctgcaatatg cacacactta gaagtctgct tcatgtattc agacttccat 180

ttcattgacg aacgaggcga atcaataatt gtggaatctg gtgatccgaa tgcattgttg 240

aaacaccggt ttgaaataat tgaaggaaga gaccgagcaa tggcctggac agtggtgaat 300

agcatctgca acaccacagg agtcgataaa cccaaatttc ttccggatct atacgactac 360

aaggaaaacc gattcactga aattggtgtg acacggaggg aagttcatat atattactta 420

gagaaagcta acaagataaa atccgaggaa acacatatcc acatcttctc attcactgga 480

gaagaaatgg ccactaaagc tgactacacc cttgatgaag agagcagggc aagaatcaaa 540

accagactat tcaccataag acaggaaatg gcaagcaggg gtctatggga ctcctttcgt 600

cagtccgaga gaggcgaaga gacaattgaa gaaagatttg aaatcacagg gaccatgcgt 660

aggcttgccg accaaagtct cccacctaac ttctccagcc ttgaaaactt tagagcctat 720

gtggatggat ttaaaccgaa cggctgcatt gagggcaagc tttctcaaat gtcgaaagaa 780

gtgaacgcca gaattgagcc atttctgaag acaacaccac gtcctctcag attgcctgat 840

ggacctccct gctcccagcg gtcgaaattc ttgctgatgg atgctctgaa attaagcatt 900

gaggacccga gccatgaggg ggaggggata ccgctatatg atgcgatcaa atgcatgaaa 960

acattctttg gctggagaga gcccaacatc atcaaaccac acgaaaaagg cataaatcca 1020

aattatctcc tggcttggaa gcaggtgctg gcagaactcc aggatattga aaatgaggat 1080

aaaatcccaa aaacaaagaa catgaagaaa acaagccaat taatgtgggc actcggagag 1140

aatatggcac cggaaaaagt ggactttgag gattgcaaag acattgacga tctgaaacag 1200

tatcacagtg atgagccaga gctcagatcg ctagcaagct ggatccagaa tgagttcaac 1260

aaggcatgtg aattgaccga ttcgagctgg atagaacttg atgagatagg ggaagatgtt 1320

gccccaattg agcacattgc aagtatgaga aggaactact tcacagcgga ggtgtcccat 1380

tgcagggcta ctgagtacat aatgaagggg gtttacataa acacagcttt gctcaatgca 1440

tcttgtgcag ccatggatga cttccaactg attccaatga taagcaaatg cagaacaaaa 1500

gaaggaagga ggaggacaaa cctgtatggg ttcattgtaa aaggaaggtc ccatttgaga 1560

aatgatactg acgtggtgaa ctttgtgagt atggaattct cccttactga cccaaggctg 1620

gagccacaca aatgggaaaa gtactgtgtt cttgaaatag gggaaatgct cttgcggact 1680

gcaataggtc aagtgtcaag gcccatgttc ctgtatgtga gaactaacgg aacctcaaaa 1740

attaagatga aatgggggat ggaaatgagg cgctgccttc ttcaatctct tcaacagatt 1800

gagagcatga tcgaggctga gtcttctatc aaagagaaag acatgaccaa agagttcttt 1860

gaaaacagat cggagacatg gccaattgga gagtcaccta agggagtgga ggaaggctcc 1920

atcgggaagg tgtgcagaac cttactagca aaatctgtgt tcaacagcct atattcatct 1980

ccacaactcg aaggattttc agctgaatcg agaaaactac tactcattgt tcaagcactt 2040

agggacaacc tggaacctgg aaccttcgat cttgaagggc tatatggagc aattgaggag 2100

tgcctgatta atgatccctg ggttttgctt aatgcatctt ggttcaactc cttcctcaca 2160

catgcactaa gatagttgtg gcaatgctac tatttgctat ccatactgtc caaaaaagta 2220

ccttgtttct act 2233

<210> 12
<211> 54
<212> DNA
<213> Artificial

<220>
<223> siDNA against Influenza A

<400> 12
tttgttactt tctcataccc ctccttttta ggtcggggtt gagaatcgtt tttt 54

<210> 13
<211> 54
<212> DNA
<213> Artificial

<220>
<223> siDNA against Influenza A

<400> 13
ttttacttta ccccttacct ctacttttta gttcagggta ggggttttcg tttt 54

<210> 14
<211> 54
<212> DNA
<213> Artificial

<220>
<223> siDNA against Influenza A

<400> 14
atacttcgtt aactcctcac ggacttttta gtttgtgtcg agttattctt cgtg 54

<210> 15
<211> 54
<212> DNA
<213> Artificial

<220>
<223> siDNA against Influenza A

<400> 15
gtaaacttac ctacagttag gctggttttc cattctattt gtaggctttc ggtt 54

## Description

Silencing of Influenza Virus RNA can be achieved by siDNA. These are oligodeoxynucleotides consisting of an antisense-strand homologous to the viral RNA and a second strand, partially complementary to the antisense-strand. The two strands are preferentially linked by a linker (eg 4 thymidines). Triple-helix formation between siDNA and the target RNA is a preferred effect. The siDNA is superior to siRNA because the formation of RNA-DNA hybrids is preferred over double-stranded DNA or double-stranded RNA, which forms as tertiary structure in RNA genomes. Also the induction of interferon is less likely. Furthermore, siDNA is effective at earlier time-points after addition to the cell than siRNA. Therefore viral RNA is targeted by siDNA before it is amplified, while siRNA targets the about 1000fold higher viral RNA population of the progeny. siDNA is easier to synthesize and is more stable. It can be combined with other siDNAs targeted against various strains in a cocktail; it can also be combined with siRNA to target early and late steps in viral replication. siDNA can be targeted to any virus strain, including the present avian pandemic strain.

### Background of the invention

Influenza virus is a member of the Orthomyxoviruses causing wide-spread infection in the human respiratory tract, but existing vaccines and drug therapy are of limited value. In a typical year 20% of the human population is afflicted by the virus, resulting in 40.000 deaths. In one of the most devastating human catastrophies in history, at least 20 million people died worldwide during the 1918 Influenza A virus pandemic. The threat of a new influenza pandemic persists because existing vaccines or therapies are of limited value. In elderly the efficacy of vaccination is only about 40%. The existing vaccines have to be redesigned every year, because of genetic variation of the viral antigens, the Haemagglutinin HA and the Neuraminidase N. Four antiviral drugs have been approved in the United States for treatment and/or prophylaxis of Influenza. However, their use is limited because of severe side effects and the possible emergence of resistant viruses.

The siDNA, the basis for his invention, is a partially double-stranded hairpin-loop structured DNA oligonucleotide of about 20 to 50 nucleotides in length, whereby the antisense strand is fully homologous to the target viral RNA and the second strand only partially homologous to the antisense strand, forming a hairpin-loop structure. It forms an RNA-DNA hybrid with the antisense strand targeting the viral RNA. The siDNA may form triple-helix with the target RNA. An RNA-DNA hybrid is thermodynamically favored over double-stranded DNA; therefore the siDNA interacting with the viral RNA is preferred over RNA-RNA interactions.

The siDNA is highly sequence-specific, length-dependent and induces cleavage (silencing) of a target RNA by an RNase H (Moelling et al, FEBS Letters, 580, 3545-3550 (2006)), which is a component in some viruses but also in the cell. RNases H removes RNA primers required for the initiation of DNA synthesis or DNA replication in the nucleus or mitochondria, but they are also present in the cytoplasm.

siDNA is related to siRNA, whereby the cleavage enzyme is highly related to the RNase H.

Recently RNA interference has been used, whereby a short double-stranded RNA, 21 to 25 nucleotides in length, siRNA, directs sequence-specific degradation of messenger mRNA. Synthetic siRNA confers transient interference of gene-expression in a sequence-specific manner. This has significant medical applications. Inhibition of Influenza virus replication by siRNA against the viral RNA encoding the nucleocapsid or one component of the polymerase, the RNA transcriptase (PA), holds promise as prophylaxis or therapy against influenza virus infection in humans (Ge et al, PNAS 100, 2718-1723 (2003)).

It was recently demonstrated by the inventor the use of siDNA targeted to the HIV genome. This activates the RNase H of the virus and leads to silencing of the viral RNA (Matzen et al, Nature Biotechnol. 25, 669-674 (2007)). The RNase H is a Hybrid-specific RNase which cleaves RNA in an RNA-DNA hybrid and was discovered by Moelling et al (Nature New Biology 234, 240-243 (1971)). The inventor also observed that cellular RNase H-like activities such as RNase H1 and RNase H2A, B, C and even Agonaute 2 contribute to siDNA-mediated silencing. Agonaute 2 (Ago2) is the enzyme known to induce siRNA-mediated silencing. The inventor showed that Ago2 is enzymatically related to RNases H and can induce siDNA-mediated silencing as well (Moelling et al. Cold Spring Harb. Symp. Quant. Biol. 71, 365-368 (2006), Small Regulatory RNAs).

Furthermore, it could be shown by the inventor that siDNA is able to induce silencing of an oncogenic retrovirus, the Spleen Focus-Forming Virus and prevent infection, cause delay of disease progression and lead to increased survival time (Nature Biotechnol. 25, 669-674 (2007)). Furthermore the inventor showed in several cases that siDNA is superior to a single-stranded antisense effect (Jendis et al. AIDS Research and Human Retroviruses 12, 1161-1168 (1996), AIDS Research and Human Retroviruses 14, 999 - 1005, (1998), Moelling et al, FEBS Letters, 580, 3545-3550 (2006), Matzen et al. Nature Biotechnol. 25, 669-674 (2007)).

Thus the effect of siDNA has been demonstrated in several cases.

The problem to be solved with the present invention is to present an effective antiviral therapeutic against Influenza virus infections by siDNA.

The problem of the invention is solved by the features of the independent claims.

An object of the invention is therefore to present siDNA oligonucleotides, capable of binding to one or more RNA target regions of Influenza virus, as antiviral therapeutic, whereby the siDNA oligonucleotides are consisting of an antisense-strand homologous to the viral RNA target and a second strand, partially complementary to the antisense-strand.

Another object of the invention is therefore to present siDNA oligonucleotides, capable of binding to one or more RNA target regions of Influenza virus, as antiviral therapeutic, whereby the siDNA oligonucleotides are consisting of an antisense-strand fully or almost fully homologous to the viral RNA target and a second strand, partially complementary to the antisense-strand forming a partially double stranded hairpin-loop-structured oligonucleotide, comprising G-clusters consisting of at least two G nucleotides in succession to allow tetrade or tetramer formation or tetra-helices or higher-ordered structures within the same siDNA oligonucleotide molecule (*cis* conformation) or through interaction with another siDNA oligonucleotide molecule (*trans* conformation).

Another object of the invention is to provide a method, respectively the use of siDNA oligonucleotides, or a combination of two or three siDNAs, as antiviral therapeutic for the treatment of Influenza virus infections, capable of binding to RNA target regions of Influenza or different Influenza virus variants, whereby different siDNA oligonucleotides may be combined in a cocktail as pharmaceutical agent.

A further object of the invention is to provide a pharmaceutical composition as antiviral therapeutic for the treatment of Influenza virus infections, comprising said siDNA oligonucleotides capable of binding to RNA target regions of Influenza virus as antiviral therapeutic.

### Summary of the invention

Therefore, siDNA oligonucleotides, capable of binding to one or more RNA target regions of Influenza virus are claimed as antiviral therapeutic, whereby the siDNA oligonucleotides are consisting of an antisense-strand homologous to the viral RNA target and a second strand, partially complementary to the antisense-strand forming a hairpin-loop structure. The siDNA oligonucleotides correspond to one or more Influenza virus target regions of at least 20 nucleotides in length, whereby the antisense strand of siDNA is fully or almost fully (more than 80%, more preferably more than 90%) homologous to the target Influenza viral RNA. Conserved target RNA regions are preferred. Further, the second strand of the siDNA oligonucleotides is connected to the antisense strand through a thymidine linker, preferred - but not exclusively - 4 nucleotides in length, and whereby further the second strand is partially (40 - 60% homology) complementary to the antisense-strand and may be able to form triple helices by non-Watson-Crick base pairing with the viral RNA target strand.

Object of the invention is the use of siDNA as antiviral therapeutic. These are oligodeoxynucleotides consisting of an antisense-strand homologous (more than 80%, more preferably more than 90%) to the viral RNA and a second strand, partially (40-60%) complementary to the antisense-strand. The siDNA is targeted to a conserved region of Influenza RNA, 20 to 25 nucleotides or longer, which is fully or almost fully homologous to the target RNA. A second strand is connected to the antisense strand through a Thymidine linker, e.g. 4 nucleotides in length. The second strand is partially complementary to the antisense-strand and may be able to form triple helices by non-Watson-Crick base pairing.

Also a preferred embodiment of the invention is the use of a combination of two or three siDNAs as described herein as antiviral therapeutic capable of binding to RNA target regions of Influenza virus as pharmaceutical agent. Further preferred is the use of a cocktail of different siDNA oligonucleotides to target different Influenza virus variants.

Influenza virus in the meaning of this invention is not restricted to Influenza A virus, Influenza B virus or Influenza C virus, but also to any virulent human pathogens of Influenza types. Therefore, other Influenza viruses such as avian Influenza virus are also an object of the invention. The serotypes that have been confirmed in humans, ordered by the number of known human pandemic deaths, are:
- H1N1 caused "Spanish Flu."
- H2N2 caused "Asian Flu."
- H3N2 caused "Hong Kong Flu."
- H5N1 is a pandemic threat in 2006-7 flu season.
- H7N7 has unusual zoonotic potential.
- H1 N2 is endemic in humans and pigs.
- H9N2, H7N2, H7N3, H10N7.

The siDNA will be applied to an infected cell or an infected individual with a transducing agent, whereby as transducing agent is selected from the following group: the virus itself, a replicating Influenza virus particle, which carries the siDNA into the cell during the process of infection, a liposome, transmembrane carriers, peptides. Most preferred is a cell transfection by using a lipid-based transfection reagent like Lullaby which is known from siRNA silencing. Other lipid based transducing agents are preferred, too.

The siDNA oligonucleotides according to the invention are preferred as antiviral therapeutic as part of a pharmaceutical composition comprising these siDNA oligonucleotides capable of binding to RNA target regions of Influenza virus or its variants.

The preferred target site on the viral RNA is highly or at least somehow conserved. Targeting a subunit of the transcriptase and its silencing would reduce viral RNA replication efficiently.

It is an unexpected unique embodiment of the invention that it acts early on the virus replication cycle and holds promise as a prevention of infection. It could be applied as nasal spray and prevent virus propagation in the body, the effect called viremia. This cannot be accomplished by siRNA.

siDNA can also be targeted to cellular mRNAs coding for proteins essential for virus replication and indirectly prevent Influenza virus replication.

### More detailed description of the invention

Under the term "homology" the following should be understood: A siDNA oligonucleotide according to the invention is consisting of an antisense-strand which is more than 80% homologous to the viral RNA target and a second strand, which is partially complementary to the antisense-strand forming a partially double stranded hairpin-loop-structure. The term "partially complementary" means a homology between 40 and 60% within the hairpin-loop.

Under the term "G-cluster" the following should be understood:

"G-cluster" according to the invention are motifs within a sequence of a siDNA oligonucleotide. A G-cluster is defined by at least 2 G nucleotides in succession ("GG"). Several G-clusters lead to tetrade or tetramer formation or tetra-helices or higher-ordered structures within the siDNA oligonucleotide molecule. Each G-cluster is separated from another G-Cluster by 1 to 20 X nucleotides, wherein X is A, T, C or a single G, followed by A, T or C. General examples (among others) according to said definition are
i. GG
ii. GGG
iii. GGXG
iv. GGGG
v. GGXXXGGXXXXGG
vi. GGXXGGXXXXGGG

G-tetrade formation may be beneficial consisting e.g. of GGXXGGXXXXGGG etc, whereby X represents basically other nucleotides than "GG", such as A, T or C or a single G, as long as the single G is followed by A, T or C. The siDNA oligonucleotides according to the invention contain G clusters to allow tetrade or tetramer formation or tetra-helices by those sequences. The hairpin-loop structure may consist preferably of up to 9 non-self-complementary sequences at the 3'- and 5'-ends, followed by 6base-pairing, 2non-pairing, 3pairing, 2non-pairing, 3pairing sequences, followed by 4Ts (see for proof of principle Moelling et al, FEBS Letters, 580, 3545-3550 (2006)). A homology of 40 to 60 % is preferred.

The tetrade or tetramer formation or tetra-helices or higher-ordered structures within the siDNA oligonucleotide molecule results of an interaction of G-clusters within the same siDNA oligonucleotide molecule (so-called *cis* conformation) or through interaction with another siDNA oligonucleotide molecule (so-called *trans* conformation). Both conformations (*cis* or *trans*) are possible.

Specific examples according to the invention are (framed sequence motifs are able to tetrade or tetramer formation or tetra-helices or higher-ordered structures; bold nucleotides indicate G-cluster) :

ODN IPA (= SEQ ID NO 12):

3'-TTTGTTACTTTCTCATACCCCTCCTTTTTAGGTCGGGGTTGAGAATCGTTTTTT-5'

ODN IPA-PPT (= SEQ ID NO 13)

3'-TTTTACTTTACCCCTTACCTCTACTTTTTAGTTCAGGGTAGGGGTTTTCGTTTT-5'

ODN IPAsi (= SEQ ID NO 14)

3′-ATACTTCGTTAACTCCTCACGGACTTTTTAGTTTGTGTCGAGTTATTCTTCGTG-5′

ODN PB-AUG (= SEQ ID NO 15)

3'-GTAAACTTACCTACAGTTAGGCTGGTTTTCCATTCTATTTGTAGGCTTTCGGTT-5'

The invention is based on the cognition that a DNA strand has a thermodynamic preference to form an RNA-DNA hybrid over double-stranded DNA or double-stranded RNA. Thus the invention is based on the not expected result that siDNA is of high preference (in contrast to siRNA) to form RNA-DNA hybrids; siDNA is therefore of advantage and a preferred object of the invention. Furthermore siDNA acts against the newly infecting incoming viral RNA, 2 to 3 days before siRNA. Thus a virus infection can be prevented by siDNA only. In contrast thereto, interferon-induction is a risk for siRNA and reduced or absent for an RNA-DNA hybrid or double-stranded DNA. Furthermore, siDNA is preferred due to the fact that DNA is easier to synthesize and more stable. Also, it can be combined with siRNA.

The siDNA may also be a chimeric molecule, consisting of ribonucleotides and desoxyribonucleotides. However, the RNase H cleavage site needs to consist of a local RNA-DNA hybrid.

siDNA is also superior to a simple antisense Oligodeoxynucleotide, because it is more stable during uptake and inside the cell and therefore more effective. It acts earlier than antisense DNA, and can target incoming RNA, while antisense DNA targets mRNA (Jekerle, V. et. al. J. Pharm. Sci. 8, 516-527 (2005)).

It was shown before that siDNA is targeting viral RNA before replication while siRNA is effective only late during replication targeting the mRNA (Westerhout, E. M. ter Brake, O. and Berkhout, B. Retrovirology 3,57-65 (2006)).

The invention takes advantage of this effect in silencing of Influenza Virus RNA. Using siDNA oligonucleotides is significantly more effective early during infection in contrast to siRNA. An unexpected advantage in conjunction with the present invention of siDNA *versus* siRNA is the targeting of incoming viral RNA before viral replication while siRNA is effective only late during replication targeting the mRNA. At this stage the RNA is highly amplified (e.g.1000fold or more) while the incoming RNA consists of a single copy. Viral RNA is destroyed within about 8 to14 hours post infection by a 5- to 10fold reduction. At this time point the siRNA effect is almost undetectable. The siRNA needs to enter a multi-protein complex, RISC, to prepare the cleavage, a process which takes about 3 days. By then the virus has replicated and spread through the organism efficiently. After 30 to 40 hours the reduction of viral RNA is similar in both cases, amounting to about 3- to 4fold reduction.

The RNA silencing inside the cell is achieved by the cellular RNases H, such as RNase H1 or RNase H2a, b, c. Also the Ago2, the siRNA-inducing silencing enzyme, is RNase H-like. These enzymes are located mainly in the nucleus but also in the cytoplasm. They are mainly responsible for removing RNA primers during DNA replication in the cell. The siDNA will lead to silencing of the viral RNA during early stages of replication and/or mRNA during late stages of replication, which requires higher doses. Treatment is - not limited to - performed by topical application, cream, spray, drops or injection of siDNA into the blood stream or intraperitoneally. Depending on the stability of the compound the treatment can be repeated. Toxicity has not been detected in mouse studies (Matzen et al. Nature Biotech. 25,669-674(2007)).

As stated above, it could be shown by the inventor that siDNA is able to induce silencing of an oncogenic retrovirus, the Spleen Focus-Forming Virus and prevent infection, cause delay of disease progression and lead to increased survival time (Nature Biotechnology 25, 669-674 (2007)). The mechanism was due to the action of the viral RNase H. A contribution of cellular RNases H or related cellular enzymes is likely. New unpublished results show that a reporter plasmid can be targeted by a siDNA and that the gene expression is silenced by RNase H1 or RNase H2a. This was demonstrated by inactivation of these RNases H, which strongly reduces the silencing effects.

### Preferred embodiments of the invention are described as follows:

Similar to siRNA a combination of two or three siDNAs may be targeted to different regions of the RNA genome or a cocktail may be designed to target different Influenza Virus variants. These include avian strains as e.g. the present avian pandemic strain H5N1. The preferred siDNA sequences are connected by a linker, preferably a thymidine linker, in particular a T4-linker.

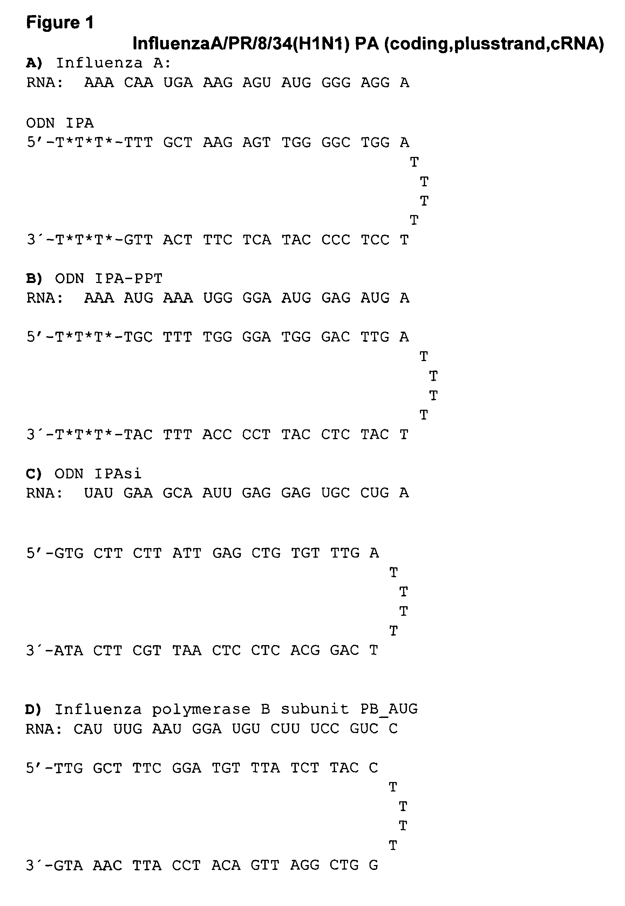

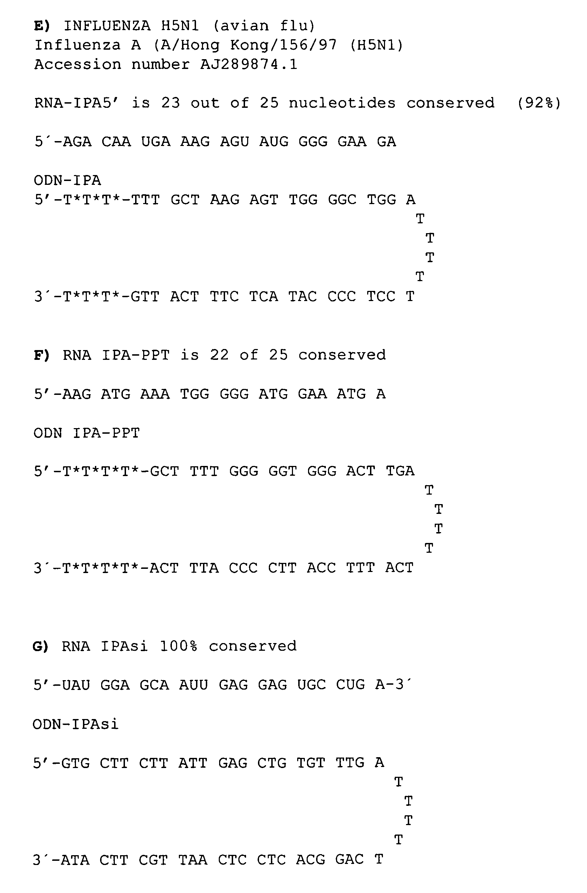

Preferred according to the invention are the sequences for siDNA as shown in figure 1, which are also an object of the invention and listed below:

| Sequence | Sequence name | Sequence |
|---|---|---|
| SEQ ID NO 1 | Target Sequence (RNA) | 5'-AAACAAUGAAAGAGUAUGGGGAGG-3' |
| SEQ ID NO 2 | ODN IPA5' (siDNA) - antisense | 5'-TTTTTTGCTAAGAGTTGGGGCTGGA-3' |
| SEQ ID NO 3 | ODN IPA5' (siDNA) - second strand | 3'-TTTGTTACTTTCTCATACCCCTCCT-5' |
| SEQ ID NO 4 | Target Sequence PPT (RNA) | 5'-AAAAUGAAAUGGGGAAUGGAGAUGA-3' |
| SEQ ID NO 5 | ODN IPA-PPT (siDNA) - antisense | 5'-TTTTGCTTTTGGGGATGGGACTTGA-3' |
| SEQ ID NO 6 | ODN IPA-PPT (siDNA) - second strand | 3-TTTTACTTTACCCCTTACCTCTACT-5' |
| SEQ ID NO 7 | Target Sequence si (RNA) | 5'-UAUGAAGCAAUUGAGGAGUGCCUGA-3' |
| SEQ ID NO 8 | ODN IPAsi (SiDNA)- antisense | 5'-GTGCTTCTTATTGAGCTGTGTTTGA-3' |
| SEQ ID NO 9 | ODN IPAsi (siDNA)- second strand | 3'-ATACTTCGTTAACTCCTCACGGACT-5' |
| SEQ ID NO 12 | siDNA Oligonucleotide ODN IPA | |
| SEQ ID NO 13 | siDNA Oligonucleotide ODN IPA-PPT | |
| SEQ ID NO 14 | siDNA Oligonucleotide ODN IPAsi | |
| SEQ ID NO 15 | siDNA Oligonucleotide ODN PB-AUG | |

The siDNA "antisense" strands and siDNA second strands are linked via a thymidine (T4) linker.

The siDNA may be stabilized by base modifications e.g. thioates at the ends and/or in the center. The invention is not restricted to those kinds of base modifications. Other base modifications, i.e phosphorothioates, P-DNA, sugar-phopsphate modifications, Morpholinos, amidates, 2'-OMe, 2'-F, 2'-MOE, LNA and further are also preferred without limitation to those modifications.

According to the invention in particular siDNA oligonucleotides comprising at least one sequence selected from the Group: SEQ ID NO 12, SEQ ID NO 13, SEQ ID NO 14 or SEQ ID NO 15 are preferred for the use as antiviral therapeutic for the treatment of Hepatitis C virus (HCV) infections and for a corresponding pharmaceutical composition.

### Figure

Abbreviations used are as follows:

| | |
|---|---|
| ODN IPA 5': | Oligodeoxynucleotide Influenza A Polymerase A, 5' End |
| ODN IPA -PPT: | Polypurine-rich |
| ODN IPAsi: | target side used for siRNA |
| PB-AUG: | Polymerase B subunit. start site (AUG) |

Linkers are indicated as 4 Ts, or can also be symbolized by a curved line; Linkers can also be modified by phosphothioate modifications Stars (*) indicate phosphothioate-modifications Vertical bars indicate Watson-Crick bonds
- Figure 1:: Preferred sequences according to the invention (A to G)

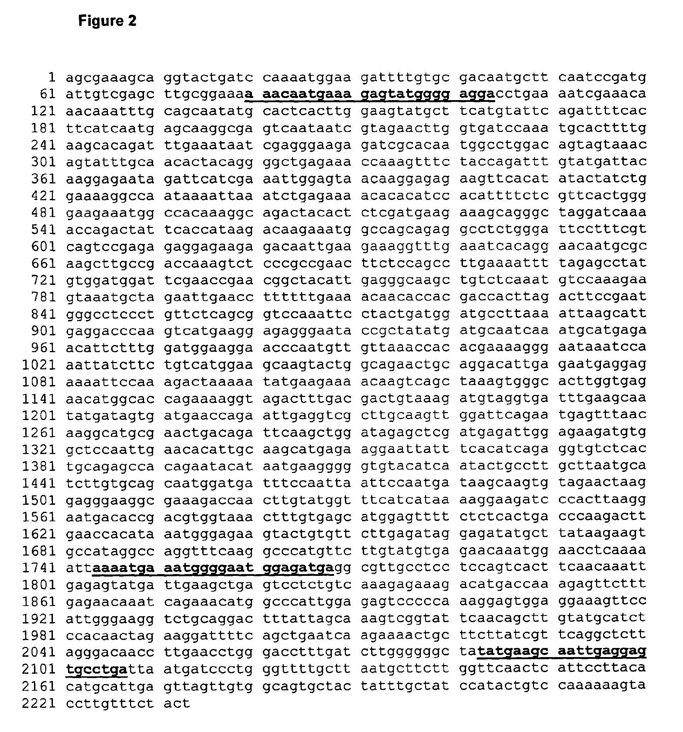

- Figure 2:: LOCUS EF467820 2233 bp cRNA linear VRL 11-MAR-2007 DEFINITION Influenza A virus (A/Puerto Rico/8/34(H1N1)) segment 3, polymerase PA, complete sequence underlined and bold are the targeted regions according to figure 1

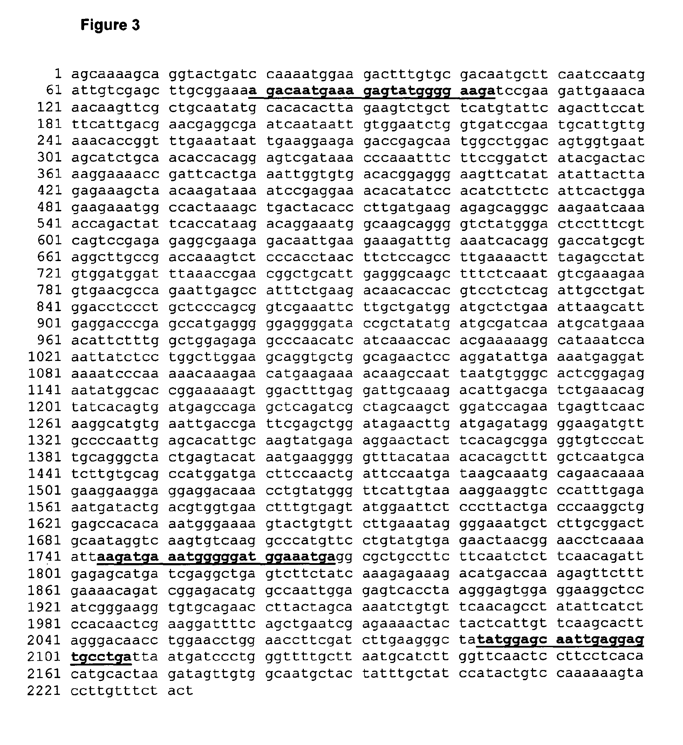

- Figure 3:: Polymerase A (PA) of Influenza H5N1 (from Pubmed (nucleotides) (A/Hong kong/156/97 (H5N1)) Accession number AJ 289874.1 underlined and bold are the targeted regions according to figure 1

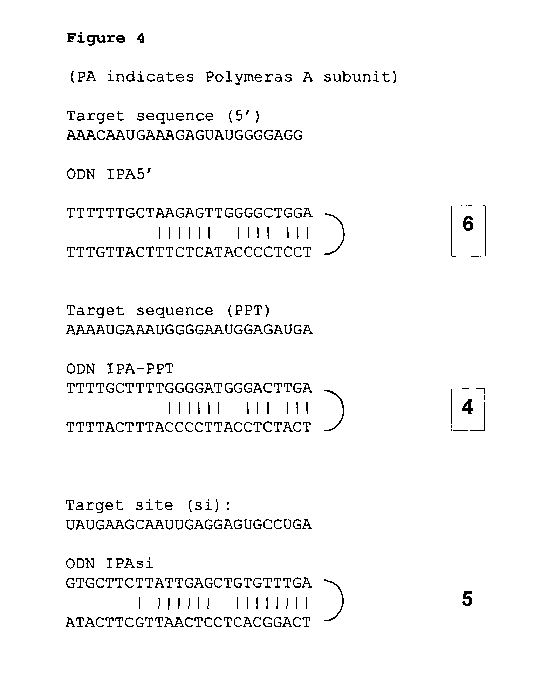

- Figure 4:: Preferred sequences according to the invention

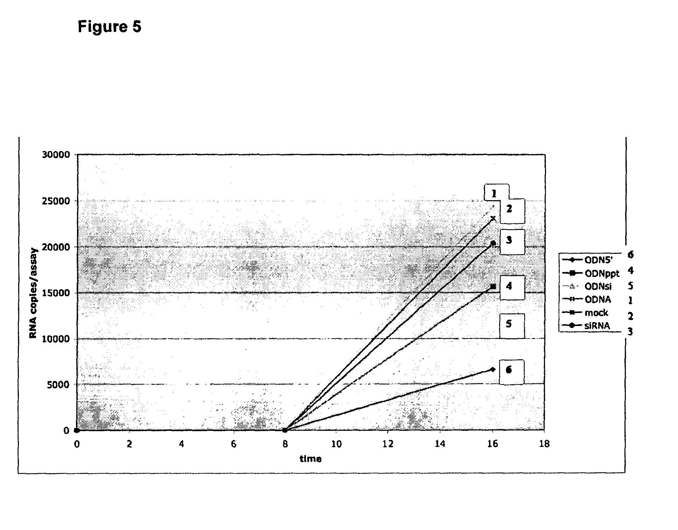

- Figure 5:: Influenza Virus was used to infect cells in tissue culture in the presence of ODNs against Influenza polymerase gene PA, three different regions (4, 5, 6, see figure 4). SiRNA was used as control and ODNA (FEBS.L. 580, 3545 (2006)) was used as an unspecific control. Lipofectamin was used to facilitate uptake. At the times indicated after infection the virus was determined in the supernatant by highly sensitive RT-PCR. Uninfected (mock-infected) cells served as another control.

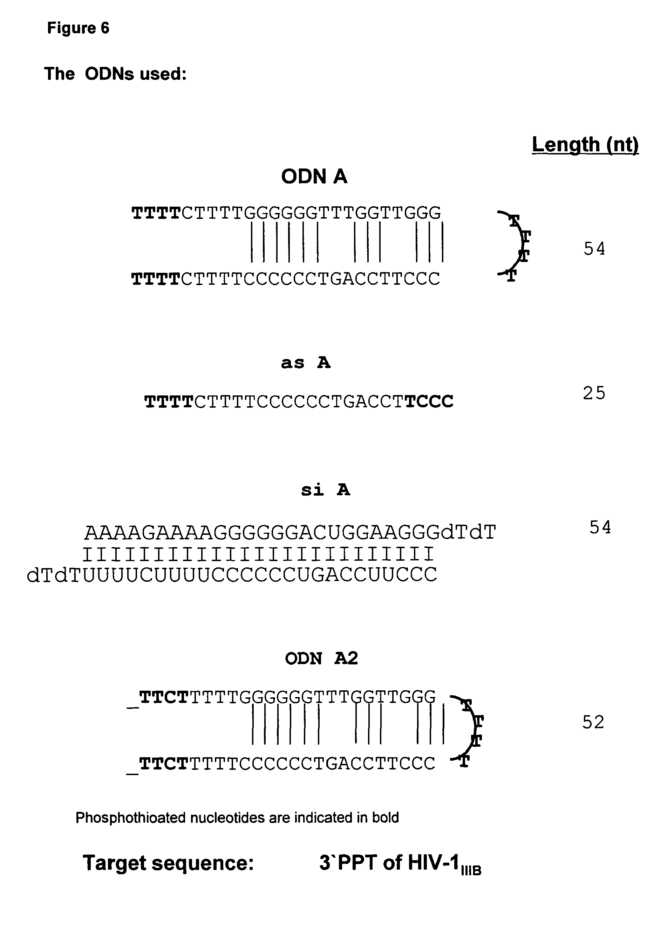

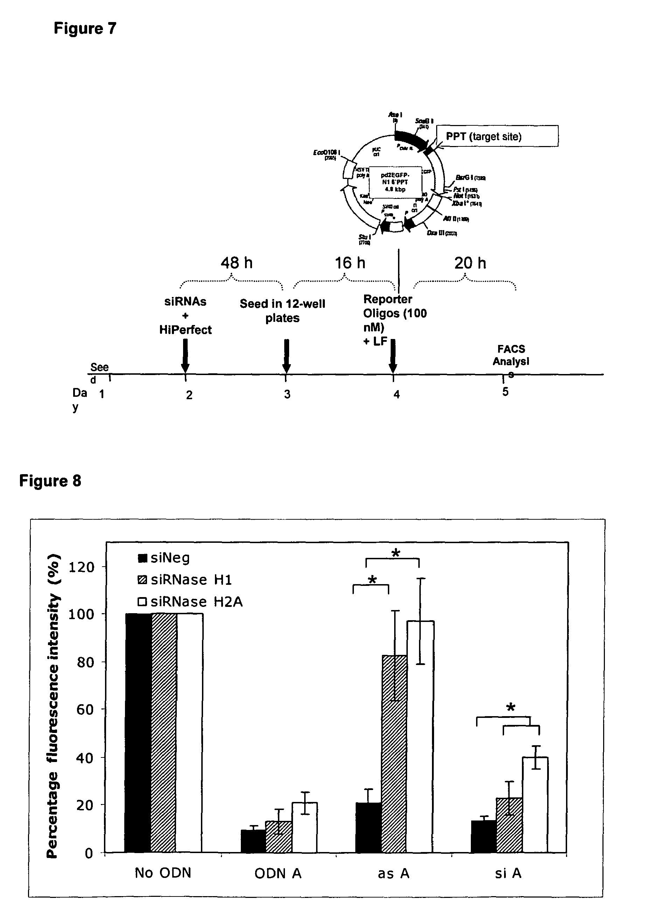

- Figure 6:: Sequences according to the experimental design of fig. 7
- Figure 7:: Determining the role of cellular RNase H1 and RNase H2A in the gene silencing effects of ODN A, asA and siA (see Fig. 3). Fig. 7 shows the experimantal design.
- Figure 8:: Knockdown of cellular RNase H1 or RNase H2A in HEK293 significantly reduced the gene silencing effects of asA but not those of ODN A or siA in live cells
HEK293 cells were transfected with siNeg, siRNase H1 or siRNase H2A siRNA duplexes (2nM) with HiPerfect for 48 h, after which the cells were trypsinised and re-seeded into 12-well plates at a density of 5x105 cells per well. After overnight incubation at 37°C, the cells were co-transfected with pEGFP-5'PPT (360 ng or 113 fmole) and
the various oligonucleotides (100 nM) using Lipofectamine2000 for 20 h before analysis by flow cytometry. The mean fluorescence intensities were normalized against those of control samples which were not treated with oligonucleotides.

## Claims

1. siDNA oligonucleotides, capable of binding to one or more RNA target regions of Influenza virus, as antiviral therapeutic, whereby the siDNA oligonucleotides are consisting of an antisense-strand homologous to the viral RNA target and a second strand, partially complementary to the antisense-strand.

2. siDNA oligonucleotides, capable of binding to one or more RNA target regions of Influenza virus, as antiviral therapeutic, whereby the siDNA oligonucleotides are consisting of an antisense-strand fully or almost fully homologous to the viral RNA target and a second strand, partially complementary to the antisense-strand forming a partially double stranded hairpin-loop-structured oligonucleotide, comprising G-clusters consisting of at least two G nucleotides in succession to allow tetrade or tetramer formation or tetra-helices or higher-ordered structures within the same siDNA oligonucleotide molecule (*cis* conformation) or through interaction with another siDNA oligonucleotide molecule (*trans* conformation).

3. siDNA oligonucleotides according to claim 1 or 2, whereby the siDNA molecule corresponds to one or more conserved Influenza target regions, of at least 20 nucleotides in length, whereby the antisense strand of siDNA is more than 80%, more preferably more than 90%. homologous to the target Influenza viral RNA

4. siDNA oligonucleotides according claim 1 or 2, whereby the second strand is connected to the antisense strand through a thymidine linker, preferred 4 nucleotides in length, and whereby the second strand is with a homology of 40 to 60% partially complementary within the hairpin-loop to the antisense-strand and may be able to form triple helices by non-Watson-Crick base pairing with the viral RNA target strand.

5. siDNA oligonucleotides according to claim 1, wherein said siDNA comprise G-clusters consisting of at least two G nucleotides in succession to allow tetrade or tetramer formation or tetra-helices or higher-ordered structures within the same siDNA oligonucleotide molecule (*cis* conformation) or through interaction with another siDNA oligonucleotide molecule (*trans* conformation).

6. siDNA oligonucleotides according to claim 2 or 5, wherein at least two the G-clusters are separated from each other by other nucleotides like A, T, C or G.

7. siDNA oligonucleotides according to one or more of the preceding claims, wherein the siDNA is stabilized by base modifications.

8. siDNA oligonucleotide according to one or more of the preceding claims, selected from the group of sequences SEQ ID NO 12, SEQ ID NO 13, SEQ ID NO 14 or SEQ ID NO 15.

9. Use of siDNA, or a combination of two or three siDNAs according to one or more of the preceding claims, as antiviral therapeutic for the treatment of Influenza virus infections, capable of binding to RNA target regions of Influenza or different Influenza virus variants, whereby different siDNA oligonucleotides may be combined in a cocktail as pharmaceutical agent.

10. Use according to claim 9, wherein the siDNA can also contain ribonucleotides, siDNA/RNA chimeras, or can be combined with siRNAs.

11. Use according to claim 9 or 10, wherein the siDNA will be applied to an infected cell or an infected individual with a transducing agent.

12. Use according to claim 11, whereby the transducing agent is selected from the following group: the virus itself, a replicating Influenza virus particle which carries the siDNA into the cell during the process of infection, a liposome, transmembrane carriers or peptides.

13. Pharmaceutical composition as antiviral therapeutic for the treatment of Influenza virus infections comprising siDNA oligonucleotides according to one or more of the preceding claims, wherein said siDNA oligonucleotides are capable of binding to RNA target regions of Influenza virus.

14. Pharmaceutical composition according to claim 13, comprising at least one sequence selected from the Group: SEQ ID NO 12, SEQ ID NO 13, SEQ ID NO 14 or SEQ ID NO 15.
